# EUROPEAN PATENT APPLICATION

(11) **EP 4 249 015 A1**
(43) Date of publication of application: **27.09.2023**
(21) Application number: 22164479.2
(22) Date of filing: 25.03.2022
(51) Int. Cl.: A61M 5/178, A61M 5/00

(54) **METHOD FOR PREPARING A STERILISED DRUG DELIVERY DEVICE**

(71) Applicant: STADA ARZNEIMITTEL AG, 61118 Bad Vilbel (DE); Xbrane Biopharma AB, 171 65 Solna (SE)
(72) Inventor: BELDA DE LAMA, Oscar, 117 60 Stockholm (SE); BASHIRI, Siavash, 117 57 Stockholm (SE); WALLSTRÖM, Anders, 135 69 Tyresö (SE); BJÖRNESTEDT, Robert, 122 32 Enskede (SE); RAMBERG, Veronica, 178 51 Ekerö (SE); VOGT, Andreas, 170 75 Solna (SE)
(74) Representative: Bergenstråhle & Partners AB

(57) **Abstract**

A method for preparing a sterilised drug delivery device (1) comprising a fluid medication, the method comprising the steps of: providing a prefilled barrel (10) containing the fluid medication, the barrel being sealed with a stopper; placing the prefilled barrel, a plunger rod (20) and a finger grip (30) in a package such that the prefilled barrel, the plunger rod and the finger grip are separated from each other, wherein one side of the package is formed by a gas-permeable sheet (40); closing and sealing the package; and exposing the sealed package to a sterilant gas under conditions such that external surfaces of the prefilled barrel, the plunger rod and the finger grip are sterilised.

## Description

### Technical Field

The present invention generally relates to a drug delivery device. More particularly, the invention relates to a method for preparing a sterilised drug delivery device containing a fluid medication.

### Background Art

Syringes are medical delivery devices used to administer a drug in liquid form to a patient. Syringes can be either prefilled, wherein a fixed dose of the drug is already provided therein, or empty and intended to be filled from a vial before administration.

Syringes typically comprise a plunger or piston that fits tightly within a cylindrical tube called a barrel. The plunger can be depressed into the barrel to expel a fluid through a distal opening. The opening can be fitted with a hypodermic needle, a nozzle or tube to guide the expelled fluid out of the opening for delivery, e.g. into body tissue.

A flange may be provided at the proximal end of the barrel as a form of finger rest to facilitate manipulation of the device. To further improve grip, usability, and ergonomic design of the syringe, a backstop or finger grip may be connected to the flange. The finger grip may have a larger dimension than the flange to increase the grip surface.

Proper sterilisation of the syringe components is critical to drug integrity and patient safety. Sterilisation may be carried out during one or more steps of manufacture/assembly. For instance, the empty components (barrel and/or plunger rod) may be sterilised before filling, and/or the assembled syringe containing the drug may be sterilised externally. External sterilisation typically occurs after the syringe has been filled, fully assembled, and placed in at least some portion(s) of its final package. Federal regulations may require external sterilisation under certain conditions and/or parameters, for some indications of use, e.g. ophthalmic indications.

There are several impediments to effective external sterilisation. The drug may be sensitive to heat, gases or radiation used in sterilisation. Additionally, a sufficient level of sterilisation may be difficult to achieve depending on the structure of the syringe. For example, surface interactions between various components of the syringe and/or the package may introduce areas that may not be sterilised effectively and/or completely. Sterilant gas may be prevented from reaching an area, which then may not be effectively sterilised. Further, some sterilant gases may be absorbed by many types of plastics and rubbers, which may negatively impact the drug product inside. Other sterilant gases may cause discoloration on the surface of the syringe, which is undesirable. In summary, there are several complications to consider in sterilising drug delivery devices.

EP 2 361 099 B1 and US 2021/0077645 A1 disclose methods of externally sterilising an object such as a drug delivery device using nitrogen dioxide (NO₂), the contents of which are hereby incorporated by reference. The drug delivery device in US 2021/0077645 A1 is a syringe which has been designed to introduce gaps and spaces between the components in an assembled state to permit airflow and thus minimise occluded spaces.

However, the known syringe is more complex to manufacture due to the gaps, and still does not eliminate all occluded areas. Thus, there is a need to improve the known sterilising methods to overcome the disadvantages mentioned above.

### Summary of Invention

An object of the present invention is therefore to provide an improved method for preparing a sterilised drug delivery device containing a fluid medication.

This object is achieved in a first aspect of the present invention in which there is provided a method for preparing a sterilised drug delivery device comprising a fluid medication, the method comprising the steps of:
providing a prefilled barrel containing the fluid medication, the barrel being sealed with a stopper;
placing the prefilled barrel, a plunger rod and a finger grip in a cavity formed in a package such that the prefilled barrel, the plunger rod and the finger grip are separated from each other, wherein one side of the package is formed by a gas-permeable sheet;
covering and sealing the package;
exposing the sealed package to a sterilant gas under conditions such that external surfaces of the prefilled barrel, the plunger rod and the finger grip are sterilised.

By physically separating the components of the drug delivery device prior to sterilisation, the method ensures that substantially all external surfaces of the prefilled barrel, the plunger rod and the finger grip are exposed to the sterilant gas and thus improves the sterilising effect.

In one embodiment, the package is a tray comprising a cavity formed therein, wherein the cavity comprises at least three separate compartments, and the method comprises placing each of the prefilled barrel, the plunger rod and the finger grip, respectively, in a separate compartment within the tray. By means of the at least three separate compartments, it is ensured that the components remain separated throughout the sterilisation procedure.

In one embodiment, each of the at least three compartments is adapted to the shape of the prefilled barrel, the plunger rod and the finger grip, respectively. By adapting the shapes of the compartments to the components, it is ensured that the components remain in place, substantially immobilised in the tray.

In one embodiment, the compartment housing the prefilled barrel comprises at least one narrow portion having an opening substantially equal in size to the diameter of the prefilled barrel. The narrow portion provides a snap-fit connection between the tray and the barrel, thus securing the prefilled barrel in the tray whilst minimising the contact surface between them to maximise the exposure to the sterilant gas.

In one embodiment, the at least three compartments are in fluid communication with each other. The fluid communication facilitates diffusion of the sterilant gas throughout the tray to reach all areas thereof.

In one embodiment, the gas-permeable sheet is impermeable to microbes, wherein the gas-permeable sheet is preferably made of a polymer, more preferably a plastic, most preferably high-density polyethylene (HDPE) fibres. The microbial barrier provided by the gas-permeable sheet enables the sterilant gas to pass through, whereas microbes and other contaminants are kept out, thus maintaining sterility of the drug delivery device.

In one embodiment, the fluid medication is an ophthalmic medication, preferably an ophthalmic medication comprising a VEGF antagonist, more preferably an ophthalmic medication comprising ranibizumab or a ranibizumab biosimilar. The sterilant gas is selected from the group of nitrogen dioxide, ethylene oxide and vaporised hydrogen peroxide.

The method and choice of sterilant gas provides efficient sterilisation of the drug delivery device, whilst the ophthalmic medication in the form of a VEGF antagonist, such as ranibizumab or a ranibizumab biosimilar, is not affected.

In one embodiment, the method further comprises the steps of:
placing the sealed package in a sterilisation chamber;
applying a predetermined vacuum level in the sterilisation chamber, providing a predetermined dose of NO₂ in the sterilisation chamber, and maintaining the vacuum level for a predetermined dwell time;
optionally, repeating the preceding step for at least one NO₂ exposure pulse;
purging the sterilisation chamber of substantially all of the NO₂; and
aerating the sterilisation chamber. Under these conditions, a sufficient level of sterility may be confirmed.

In one embodiment, the method comprises:
placing at least one sealed package in a container, the container being arranged to receive a plurality of sealed packages in a substantially uniform distribution such that each sealed package is secured in place for exposure to the sterilant gas, wherein at least one wall of the container is arranged to allow passage of gas;
placing the container in the sterilisation chamber. The container enables simultaneous sterilisation of plurality of drug delivery devices and also provides a suitable package for shipping and transport.

In one embodiment, the predetermined vacuum level is about 0.1-100 kPa, the predetermined dose of NO₂ in the sterilisation chamber has a concentration of about 0.1-50 mg/L, and the predetermined dwell time is at least 1 minute.

In a second aspect of the present invention, there is provided a sterilised drug delivery device comprising a fluid medication, obtained by a method according to the first aspect.

In a third aspect of the present invention, there is provided a medical package for a sterilised drug delivery device, wherein the medical package comprises a tray with a cavity formed therein, the cavity comprising at least three separate compartments, each compartment containing one of a prefilled barrel, a plunger rod and a finger grip, respectively, wherein the cavity is covered and sealed by a gas-permeable sheet to form a sealed tray, wherein the sealed tray has been exposed to a sterilant gas under conditions such that external surfaces of the prefilled barrel, the plunger rod and the finger grip are sterilised.

In one embodiment, each of the at least three separate compartments is adapted to the shape of the prefilled barrel, the plunger rod and the finger grip, respectively.

In one embodiment, the compartment housing the prefilled barrel comprises at least one narrow portion having an opening substantially equal in size to the diameter of the prefilled barrel.

In one embodiment, the at least three compartments are in fluid communication with each other.

In one embodiment, the gas-permeable sheet is impermeable to microbes, wherein the gas-permeable sheet is preferably made of a polymer, more preferably a plastic, most preferably high-density polyethylene (HDPE) fibres.

In one embodiment, the fluid medication is an ophthalmic medication, preferably an ophthalmic medication comprising a VEGF antagonist, more preferably an ophthalmic medication comprising ranibizumab or a ranibizumab biosimilar.

In a fourth aspect of the present invention, there is provided a container comprising at least one medical package according to the third aspect, wherein the container comprises means for arranging a plurality of sealed packages in a substantially uniform distribution inside the container such that each sealed package is secured in place for exposure to a sterilant gas, wherein at least one wall of the container is arranged to allow passage of gas.

In one embodiment, the means for arranging the plurality of sealed packages in a substantially uniform distribution inside the container comprises a rack, an arrangement of partition walls, or a framework structure.

In one embodiment, the means for arranging the plurality of sealed packages in a substantially uniform distribution inside the container comprises a material which minimises absorption of sterilant gas, preferably a composite or polymer, more preferably polyethylene.

### Brief Description of Drawings

The invention is now described, by way of example, with reference to the accompanying drawings, in which:
Fig. 1 shows a perspective view of an exemplary drug delivery device.
Fig. 2 shows the drug delivery device of Fig. 1 in an unassembled state.
Fig. 3 shows a package for receiving the components of the drug delivery device of Fig. 2.
Fig. 4 shows the components of the unassembled drug delivery device of Fig. 2 placed in a sealed package in accordance with one embodiment of the present invention.
Fig. 5 shows how the sealed package of Fig. 4 may be opened to access the sterilised components of the unassembled drug delivery device of Fig. 2 prior to use.

### Description of Embodiments

In the following, a detailed description of a sterilisation method according to the present invention is presented. In the drawing figures, like reference numerals designate identical or corresponding elements throughout the several figures. It will be appreciated that these figures are for illustration only and are not in any way restricting the scope of the invention.

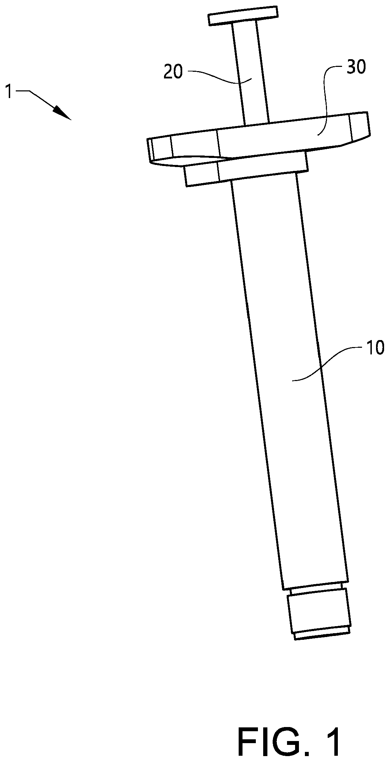

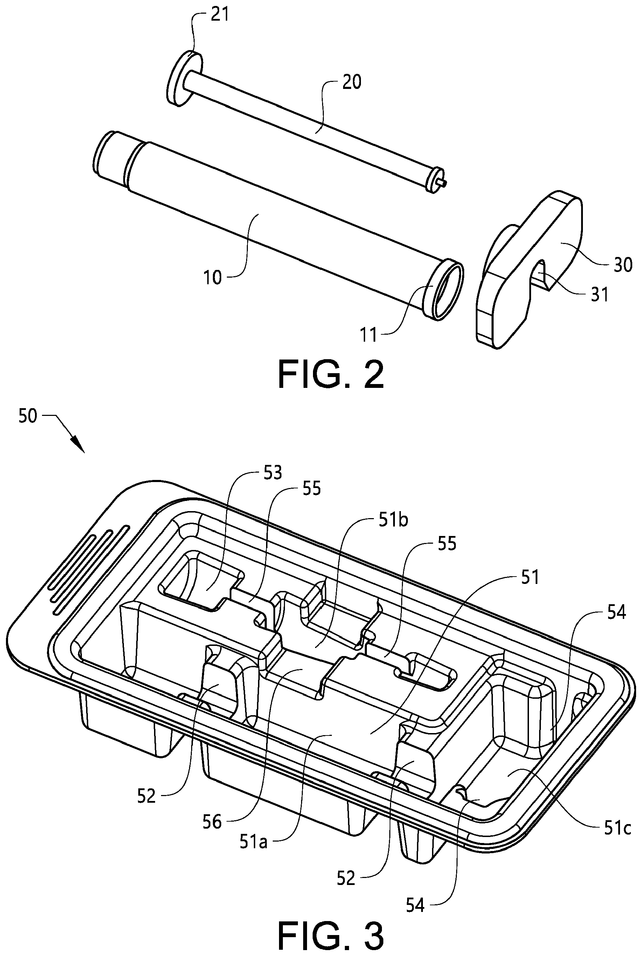

Fig. 1 is a perspective view of an exemplary drug delivery device 1 in the form of a syringe which comprises a barrel 10 filled with a fluid medication and sealed with a stopper (not shown), a plunger rod 20 and a finger grip 30. Fig. 2 shows the individual components of the syringe 1 in an unassembled state. The open proximal end of the barrel 10 comprises a flange 11 for engagement with the finger grip 30, and the distal end is arranged to mate with a piercing element (not shown). For instance, the distal end may include a Luer lock fitting with threads as known in the art. Additionally, the distal end of the barrel 10 may be covered by a protective cap (not shown).

The barrel 10 may be made of plastic, glass, or any suitable material. As a more specific example, the syringe barrel may be made of a plastic material, preferably including at least one or more of the following materials: certain grades of polypropylene (homo-polymer and/or polypropylene co-polymer), cyclic olefin copolymer (COC), cyclic olefin polymer (COP), or other suitable materials.

The flange 11 may include a diameter larger than that of the syringe barrel 10 and may serve as a finger rest permitting the user to manipulate the syringe 1 during use. For example, the user may rest two or more of her/his fingers against the flange 11 while using her/his thumb to depress the plunger rod end 21. The finger grip 30 may be coupled with the flange portion 11 to effectively extend the flange portion 11 and thereby extend the length of the grip surface. It may be desirable for a user to have a larger effective grip surface to improve grip, usability, and or ergonomic design of the syringe 1, particularly for certain applications of the syringe 1 such as ophthalmic indications. However, it may be undesirable to increase the size of the flange portion due to space constraints during manufacturing and shipping, advantages of scale for using a standard syringe/flange configuration. Additionally, syringes are commonly used in autoinjectors, which have a profile that may not accommodate a syringe with an enlarged flange size. Therefore, it may be desirable to have an additional component, such as a finger grip component 30, that is able to be attached to and/or coupled with the syringe 1 at a point in the manufacturing process or just prior to use.

The finger grip 30 may be manufactured from any suitable material. For example, it may be moulded out of polypropylene ("PP") or acrylonitrile butadiene styrene ("ABS"). ABS may have the advantage of being stiffer than PP and other materials so that the finger grip can become lighter. If the finger grip is made from PP, it may have a minimum wall thickness of 1.5 mm thickness, whereas if it is made from ABS it may have a minimum wall thickness of 1 mm. The finger grip 30 comprises a recess 31 for receiving the shaft of the plunger rod 20. The recess 31 guides the plunger rod 20 during depression to improve control of the injection of the fluid medication.

Referring now to Fig. 3, there is shown a package in accordance with one embodiment of the present invention. The package is in the form of a tray 50 with a cavity 51 formed therein, e.g. by means of a thermoforming process in which a base web made of a plastic sheet is heated and formed to the desired shape of the cavity or pocket in a mould. Generally the plastic sheet is non-collapsible to provide a rigid tray 50. The cavity 51 comprises at least three separate compartments 51a, 51b, 51c for receiving the prefilled barrel 10, the plunger rod 20, and the finger grip 30, respectively. The compartments 51a, 51b, 51c are in fluid communication with each other so as to allow passage of gas between them.

In other embodiments, the package may be in the form of a pouch, e.g. formed by two opposing sheets sealed around the edges to form a cavity therebetween and leave an opening.

In one embodiment, the compartments 51a, 51b, 51c in the tray are adapted to the shape of the barrel 10, the plunger rod 20 and the finger grip 30, respectively. The different adapted shapes of the compartments 51a, 51b, 51c facilitates correct placement of the respective components and helps securing the components to prevent them from moving around in the tray 50. For instance, the compartment 51b which receives the plunger rod 20 comprises a wider section 53 at one end to accommodate the plunger rod end 21. The compartment 51c which receives the finger grip 30 comprises a pair of grooves 54 to secure the edges of the finger grip 30.

In one embodiment, at least the compartment 51a which receives the barrel 10 comprises one or more narrow portions 52 formed by support walls to receive and support the barrel 10. The opening between the support walls may have a dimension which substantially corresponds to the diameter of the barrel 10 to achieve a snap-fit connection between the support walls and the barrel 10. Similarly, the compartment 51b for the plunger rod 20 may also comprise one or more narrow portions 55 which substantially correspond to the diameter of the shaft of the plunger rod 20 to achieve a snap-fit connection between the support walls and the plunger rod 20.

The package may include protrusions or spacers to limit surface area contact between the tray and the barrel, thereby reducing or preventing occluded spaces therebetween. For example, the package may include protrusions extending outward from the support walls of the package to create a gap between the barrel and the package, thereby minimising or preventing occluded spaces between the same. Thereby, the protrusions or spacers permit an effective connection between the package and the barrel while allowing sterilisation gas to flow in between the respective components during external sterilisation steps. The protrusions or spacers may also improve aeration (reduced time and/or improved effectiveness) of the sterilisation gas after the external sterilisation step.

The tray 50 may also comprise one or more indentations or recesses 56 arranged substantially perpendicular to the orientation of the compartments 51a and 51b for the barrel 10 and plunger rod 20, respectively, transecting the compartments 51a and 51b. The indentations 56 facilitate removal of the barrel 10 and plunger rod 20 from the tray 50 by providing improved access. Additionally, the indentations 56 provide increased fluid communication between the compartments 51a, 51b to improve diffusion of gas.

As another example, the package may not include a snap-fit arrangement with the syringe and may instead allow the syringe to rest therewithin to allow sterilisation gas to flow in between the respective components during external sterilisation steps. In such a design, the gaps between the support walls would be larger than the diameter of the syringe barrel to allow space between the support walls and the barrel. Also, in such a design, the package lid would preferably prevent the barrel from moving out of the package.

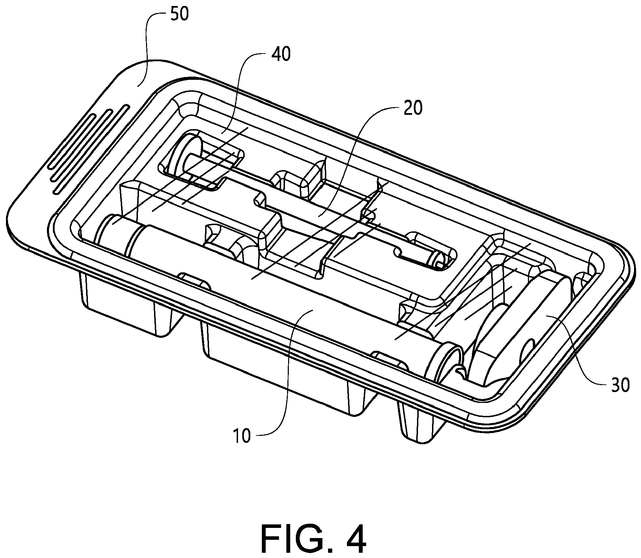

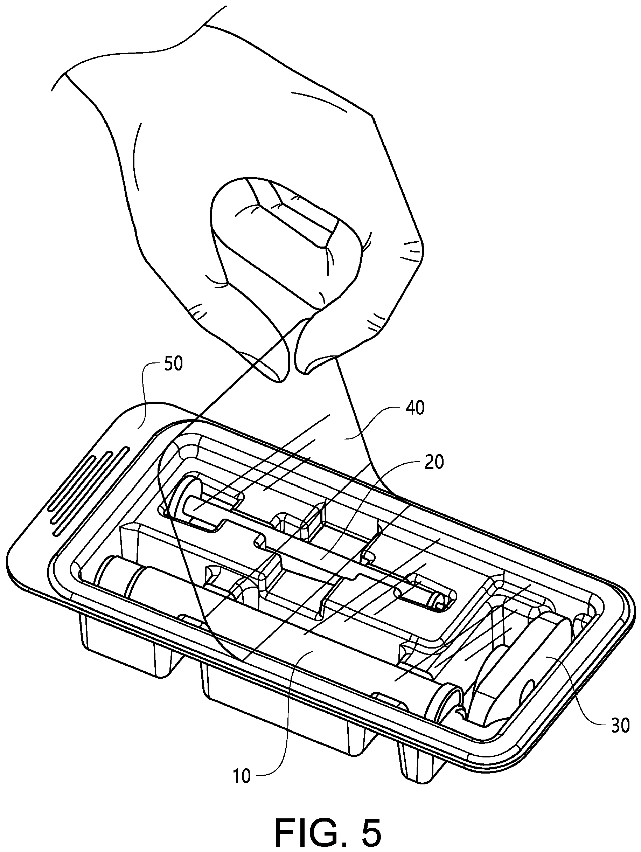

Referring now to Fig. 4, after placement of the components of the syringe 1 in the tray 50, the cavity 51 is closed and sealed by means of a lidding film or cover comprising a gas-permeable sheet 40. The gas-permeable sheet 40 allows passage of gas, such as sterilant gases used for sterilisation of the syringe 1, whilst also providing a microbial barrier to prevent contamination of the sterilised syringe 1. To this end, the gas-permeable sheet is made of a polymer, preferably a plastic. One suitable material for the gas-permeable sheet is high-density polyethylene (HDPE) fibres, also known as Tyvek^{®}, which exhibits excellent resistance to penetration by bacterial spores and other contaminating microorganisms. In the embodiment of a pouch, at least one of the two sheets is a gas-permeable sheet. Fig. 5 shows a user removing the lidding film of a sealed package prior to use of the sterilised syringe 1.

Next, the sealed package is exposed to a sterilant gas under conditions such that external surfaces of the prefilled barrel 10, the plunger rod 20 and the finger grip 30 are sterilised. Nitrogen dioxide (NO₂) gas is a rapid and effective sterilant for use against a wide range of microorganisms, including common bacteria, viruses, and spores. The unique physical properties of NO₂ gas allow for sterilant dispersion in an enclosed environment at room temperature and atmospheric pressure. The mechanism for lethality is the degradation of DNA in the spore core through nitration of the phosphate backbone, which kills the exposed organism as it absorbs NO₂. This degradation occurs at even very low concentrations of the gas. NO₂ has a boiling point of 21 °C at sea level, which results in a relatively highly saturated vapour pressure at ambient temperature. Because of this, liquid NO₂ may be used as a convenient source for the sterilant gas. Additionally, the low levels of concentration required, coupled with the high vapour pressure, assures that no condensation occurs on the devices being sterilised. NO₂ is also less corrosive than other sterilant gases and is compatible with most medical materials and adhesives. Other alternatives for the sterilant gas include ethylene oxide (EO) and vaporised hydrogen peroxide (VHP).

In one embodiment, the exposure to sterilant gas is carried out by placing the sealed package in a sterilisation chamber, applying a predetermined vacuum level in the sterilisation chamber, providing a predetermined dose of NO₂ in the sterilisation chamber, and maintaining the vacuum level for a predetermined dwell time. The vacuum level can be about 0.1-100 kPa (0.75-750 Torr), preferably about 0.5-50 kPa (3.75-375 Torr), the dose of NO₂ in the sterilisation chamber has a concentration of about 0.1-50 mg/L, preferably about 2-20 mg/L, and the dwell time can be at least 1 minute. Optionally, the preceding step is repeated for at least one NO₂ exposure pulse. Finally, the sterilisation chamber is purged of substantially all of the NO₂ and aerated. Under these conditions, a sufficient level of sterility may be confirmed.

In one embodiment, before exposure to sterilant gas in the sterilisation chamber, the sealed package is placed in a container arranged to receive a plurality of sealed packages in a substantially uniform distribution such that each sealed package is secured in place for exposure to the sterilant gas. For instance, the container may comprise a rack, an arrangement of partition walls or a framework structure to hold the sealed packages. The means for arranging the plurality of sealed packages in a substantially uniform distribution inside the container comprises a material which minimises absorption of sterilant gas, preferably a composite or polymer, more preferably polyethylene. This reduces aggregation of sterilant gas residuals in the container.

At least one wall of the container is arranged to allow passage of gas, e.g. comprising one or more apertures or a gas-permeable material. The container with the sealed package(s) is then placed in the sterilisation chamber for exposure to sterilant gas as explained above. The container enables simultaneous sterilisation of plurality of drug delivery devices and also provides a suitable package for shipping and transport. Before shipping, the container with the sterilised sealed packages may be wrapped in a film for protection.

### Examples

In order to test and compare the sterilisation method in accordance with one embodiment of the present invention to prior known methods, a study for NO₂ sterilisation was conducted for three different configurations: a) the syringe barrel, the plunger rod and the finger grip were assembled in a pouch, b) the syringe barrel, plunger rod and the finger grip were unassembled in a pouch, and c) the syringe barrel, plunger rod and the finger grip were unassembled in a 3-compartment sealed tray. The results are summarised in Table 1 below. A total of 8 different sterilisation cycles were performed, cycle 1 in configuration a), cycles 2-4 in configuration b), and cycles 5-8 in configuration c). In order to determine the sterility, the syringes were challenged at two locations: the barrel and the finger grip using a biological indicator (BI) containing *Geobacillus stearothermophilus* with a population of at least 1×10⁶ endospores.

**Table 1. Examples of NO₂ sterilisation**

| Cycle Number | Vacuum (Torr) | NO₂ Concentration in the sterilisation chamber (mg/L) | Number of sterilisation pulses | Dwell time (min) | Number of aerations | Fraction negative BI (#neg./total #) | |
|---|---|---|---|---|---|---|---|
| | | | | | | Barrel | Finger grip |
| 1 | 20 | 20 | 4 | 20 | 12 | 6/6 | 4/6 |
| 2 | 20 | 10 | 2 | 10 | 16 | 6/6 | 6/6 |
| 3 | 20 | 5 | 1 | 10 | 16 | 6/6 | 6/6 |
| 4 | 20 | 3 | 1 | 10 | 16 | 6/6 | 5/6 |
| 5 | 20 | 3 | 1 | 5 | 48 | 13/15 | 12/15 |
| 6 | 20 | 3 | 1 | 2 | 48 | 10/15 | 9/15 |
| 7 | 20 | 10 | 1 | 10 | 84 | N/A | 15/15 |
| 8 | 20 | 10 | 2 | 10 | 84 | N/A | 15/15 |

In Table 1, the "Vacuum (Torr)" column label refers to the maximum vacuum force applied on the external sterilisation chamber during sterilisation. As shown, the vacuum force is at 20 Torr (about 2.66 kPa), although different vacuum forces may be appropriate. The stronger the vacuum force, the greater the chance of demonstrating kill of the target bioburden. However, if the vacuum force becomes too high, then the process may result in undesirable effects on the medicament, such as causing the stopper in the barrel to move undesirably (i.e., to move past the sterility barrier and cause a sterility breach).

The "NO₂ Concentration (mg/L)" column refers to the concentration (in mg) of NO₂ per litre of air in the external sterilisation chamber. As shown, the concentration in Table 1 varies between 3 and 20 mg/L, although different doses may be appropriate. The higher the dose of NO₂ during sterilisation, the faster and more completely the drug delivery device will be sterilised. However, if the dose of sterilant gas becomes too high, then the process may result in undesirable effects on the medicament, such as contaminating the inside of the drug barrel with sterilant gas (i.e., ingress of sterilant gas and/or discoloration of the syringe components).

The "Number of sterilisation pulses" column refers to the number of times during the NO₂ process that the gas is injected by pulling a vacuum. As shown, the pulses for each row in Table 1 varies from 1 to 4, although different values may be appropriate. The higher the number of pulses, the greater the chance of demonstrating kill of the target bioburden. However, if the number of pulses becomes too high, then the process may result in undesirable effects on the medicament, such as contaminating the inside of the drug barrel with sterilant gas.

The "Dwell time (min)" column refers to the amount of time that the drug delivery device sits in the sterilisation chamber while sterilant gas is present. For Table 1, the total dwell time is equal to the "Dwell Time" column times the "Number of sterilisation pulses" column. For example, for the first row of Table 1, the samples would experience a total dwell time of 80 minutes. As shown, the dwell times listed in Table 1 vary between 2 and 20 minutes, although different dwell times may be appropriate. The dwell time also increases the likelihood of demonstrating kill of the target bioburden. However, if the dwell time becomes too high, then the process may result in undesirable effects on the medicament, such as contaminating the inside of the drug barrel with sterilant gas.

The column labelled "Number of Aerations" refers to the number of times that the chamber is aerated after the gas is purged from the chamber. The exemplary process may utilise aeration exchanges of 12, 16, 48, 84, or any desirable number. Up to a certain point, by increasing the number of aerations, the manufacturer may increase the likelihood that all or substantially all of the sterilant gas is removed from the syringe and package (post-purge).

The column labelled "Fraction negative BI (#neg./total #)" refers to the number of samples demonstrating lethality of the BI, i.e. kill of the target bioburden, in relation to the total number of samples challenged for the two different locations barrel and finger grip. As may be seen, cycle 1 performed in configuration a) with a relatively high NO₂ concentration of 20 mg/L, high number of pulses (4), long dwell time (20 minutes) and low number of aerations (12) still resulted in only four of six (4/6) samples reaching lethality of the BI at the finger grip location. In other words, sterility cannot be achieved to a satisfactory level when using NO₂ as sterilant gas to sterilise an assembled syringe in a pouch.

Cycles 2-4 were performed in configuration b) with the syringe barrel, the plunger rod and the finger grip unassembled in a pouch. In the unassembled state, the components of the syringe are separated from each other such that occluded areas are reduced or eliminated. In cycles 2-4, the NO₂ concentration was varied from 10 mg/L to 3 mg/L, the number of pulses was varied from 2 to 1, the dwell time was 10 minutes and the number aerations was 16. In all three cycles, lethality of the BI was achieved at the barrel in all (6/6) samples, and in the two cycles 2 and 3 with NO₂ concentrations of 10 mg/L and 5 mg/L, respectively, lethality of the BI was also achieved at the finger grip in all (6/6) samples. At the lowest NO₂ concentration of 3 mg/L in cycle 4, five of six (5/6) samples reached lethality.

Cycles 5-8 were performed in configuration c) with the syringe barrel, the plunger rod and the finger grip unassembled in a 3-compartment sealed tray. In the unassembled state, the components of the syringe are separated and kept from each other such that occluded areas are reduced or eliminated. In cycles 5-8, the NO₂ concentration was varied from 3 mg/L to 10 mg/L, the number of pulses was varied from 1 to 2, the dwell time was varied from 2 minutes to 10 minutes and the number aerations was varied from 48 to 84. In cycle 5 with a NO₂ concentration of 3 mg/L, one sterilisation pulse, dwell time of 5 minutes and 48 aerations, thirteen of fifteen (13/15) samples reached lethality of the BI at the barrel and twelve of fifteen (12/15) samples reached lethality of the BI at the finger grip. In cycle 6, the dwell time was reduced to 2 while keeping the remaining parameters identical to cycle 5. Here, 10 of fifteen (10/15) samples reached lethality of the BI at the barrel and nine of fifteen (9/15) samples reached lethality of the BI at the finger grip.

In cycles 7 and 8, the NO₂ concentration was increased to 10 mg/L, the dwell time was increased to 10 minutes and the number of aerations was increased to 84. Also in cycle 8, the number of pulses was increased to 2. In both cycles, lethality of the BI at the finger grip was achieved in all (15/15) samples.

Cycles 1-8 in Table 1 were performed using a relative humidity (% RH) of 75% in the sterilisation chamber, although different relative humidity values may be appropriate. Increasing the relative humidity also increases the likelihood of demonstrating kill of the target bioburden.

A preferred embodiment of a method for preparing a sterilised drug delivery device comprising a fluid medication according to the present invention has been described. However, the person skilled in the art realises that this can be varied within the scope of the appended claims without departing from the inventive idea.

All the described alternative embodiments above or parts of an embodiment can be freely combined without departing from the inventive idea as long as the combination is not contradictory.

## Claims

1. A method for preparing a sterilised drug delivery device (1) comprising a fluid medication, the method comprising the steps of:
a) providing a prefilled barrel (10) containing the fluid medication, the barrel being sealed with a stopper;
b) placing the prefilled barrel, a plunger rod (20) and a finger grip (30) in a package such that the prefilled barrel, the plunger rod and the finger grip are separated from each other, wherein one side of the package is formed by a gas-permeable sheet (40);
c) closing and sealing the package;
d) exposing the sealed package to a sterilant gas under conditions such that external surfaces of the prefilled barrel, the plunger rod and the finger grip are sterilised.

2. The method according to claim 1, wherein the package is a tray (50) comprising a cavity (51) formed therein, wherein the cavity comprises at least three separate compartments (51a, 51b, 51c), and step b) comprises placing each of the prefilled barrel, the plunger rod and the finger grip, respectively, in a separate compartment within the tray.

3. The method according to claim 2, wherein each of the at least three compartments is adapted to the shape of the prefilled barrel, the plunger rod and the finger grip, respectively.

4. The method according to claim 2 or 3, wherein the compartment (51a) housing the prefilled barrel comprises at least one narrow portion (52) having an opening substantially equal in size to the diameter of the prefilled barrel.

5. The method according to any one of claims 2-4, wherein the at least three compartments are in fluid communication with each other.

6. The method according to any one of the preceding claims, wherein the gas-permeable sheet is impermeable to microbes, wherein the gas-permeable sheet is preferably made of a polymer, more preferably a plastic, most preferably high-density polyethylene (HDPE) fibres.

7. The method according to any one of the preceding claims, wherein the fluid medication is an ophthalmic medication, preferably an ophthalmic medication comprising a VEGF antagonist, more preferably an ophthalmic medication comprising ranibizumab or a ranibizumab biosimilar.

8. The method according to any one of the preceding claims, wherein the sterilant gas is selected from the group of nitrogen dioxide, ethylene oxide and vaporised hydrogen peroxide.

9. The method according to claim 1, wherein step d) comprises the steps of:
i) placing the sealed package in a sterilisation chamber;
ii) applying a predetermined vacuum level in the sterilisation chamber, providing a predetermined dose of NO₂ in the sterilisation chamber, and maintaining the vacuum level for a predetermined dwell time;
iii) optionally, repeating step ii) for at least one NO₂ exposure pulse;
iv) purging the sterilisation chamber of substantially all of the NO₂; and
v) aerating the sterilisation chamber.

10. The method according to claim 9, wherein step i) comprises:
- placing at least one sealed package in a container, the container being arranged to receive a plurality of sealed packages in a substantially uniform distribution such that each sealed package is secured in place for exposure to the sterilant gas, wherein at least one wall of the container is arranged to allow passage of gas;
- placing the container in the sterilisation chamber.

11. The method according to claim 9 or 10, wherein the predetermined vacuum level is about 0.1-100 kPa, the predetermined dose of NO₂ in the sterilisation chamber has a concentration of about 0.1-50 mg/L, and the predetermined dwell time is at least 1 minute.

12. A sterilised drug delivery device comprising a fluid medication, obtained by a method according to any one of the preceding claims.

13. A medical package for a sterilised drug delivery device, wherein the medical package comprises a tray with a cavity formed therein, the cavity comprising at least three separate compartments, each compartment containing one of a prefilled barrel, a plunger rod and a finger grip, respectively, wherein the cavity is covered and sealed by a gas-permeable sheet to form a sealed tray, wherein the sealed tray has been exposed to a sterilant gas under conditions such that external surfaces of the prefilled barrel, the plunger rod and the finger grip are sterilised.

14. The medical package according to claim 13, wherein each of the at least three separate compartments is adapted to the shape of the prefilled barrel, the plunger rod and the finger grip, respectively.

15. The medical package according to claim 13 or 14, wherein the compartment housing the prefilled barrel comprises at least one narrow portion having an opening substantially equal in size to the diameter of the prefilled barrel.

16. The medical package according to any one of claims 13-15, wherein the at least three compartments are in fluid communication with each other.

17. The medical package according to any one of claims 13-16, wherein the gas-permeable sheet is impermeable to microbes, wherein the gas-permeable sheet is preferably made of a polymer, more preferably a plastic, most preferably high-density polyethylene (HDPE) fibres.

18. The medical package according to any one of claims 13-17, wherein the fluid medication is an ophthalmic medication, preferably an ophthalmic medication comprising a VEGF antagonist, more preferably an ophthalmic medication comprising ranibizumab or a ranibizumab biosimilar.

19. A container comprising at least one medical package according to any one of claims 12-17, wherein the container comprises means for arranging a plurality of sealed packages in a substantially uniform distribution inside the container such that each sealed package is secured in place for exposure to a sterilant gas, wherein at least one wall of the container is arranged to allow passage of gas.

20. The container according to claim 19, wherein the means for arranging the plurality of sealed packages in a substantially uniform distribution inside the container comprises a rack, an arrangement of partition walls, or a framework structure.

21. The container according to claim 19 or 20, wherein the means for arranging the plurality of sealed packages in a substantially uniform distribution inside the container comprises a material which minimises absorption of sterilant gas, preferably a composite or polymer, more preferably polyethylene.
